# EUROPEAN PATENT APPLICATION

(11) **EP 1 862 108 A2**
(43) Date of publication of application: **05.12.2007**
(21) Application number: 07010783.4
(22) Date of filing: 31.05.2007
(51) Int. Cl.: A61B 1/04, A61B 1/05

(54) **Optically coupled endoscope with microchip**

(30) Priority: 31.05.2006 US 444079
(71) Applicant: Karl Storz Endovision, Charlton, MA 01507 (US)
(72) Inventor: Hopkins, Vernon, Worcester, MA 01604 (US); Landry, Dana J., Sturbridge, MA 01566 (US)
(74) Representative: Heuckeroth, Volker

(57) **Abstract**

An endoscope device providing electrical isolation between the endoscope and a camera control unit coupled to the endoscope. The endoscope uses an optical channel to transmit illuminating light to the endoscope and to transmit image data generated by an imaging device to the camera control unit. In this manner, only an optical connection between the endoscope and the camera control unit exits. Alternatively, a wireless channel for transmission of the image data and any commands signals may be utilized.

## Description

### FIELD OF THE INVENTION

The invention relates to an endoscope device that provides a relatively high level of electrical isolation to a patient, and more specifically, the invention relates to and endoscope device that effectively electrically isolates the patient from connection to an electrical power source during a surgical procedure.

### BACKGROUND OF THE INVENTION

Endoscope devices have been used for minimally invasive surgical procedures for some time providing significant advantages for the patient. Endoscopes, whether a direct viewing type provided with, for example, and eye piece for the physician to look through, or video endoscopes including a camera to pick up and transmit images to a screen for viewing by the physician, allow for viewing of a surgical area. Typically, endoscopes are provided with an illuminating light source to provide illumination of the cavity for viewing by the physician.

With the advent of numerous electrical and electronic surgical devices, patient safety has become a high priority, especially with regard to electrical shock of the patient during a surgical procedure. According to IEC 601, electro-medical devices require galvanic separation between the primary electrical power supply side and the patient/user unit, which is supposed to provide for increased safety and avoidance of electrical shock of the patient. Especially is this important when high-frequency and electro-cautery devices are used.

In order to provide this electrical isolation, systems have sought to provide isolation transformers positioned between the main electrical power and the patient to effectively isolate the patient from the supply side power. Other systems has sought to position high-frequency transformers between the patient and power source. While these systems have provided a degree of electrical isolation and thereby increased safety for the patient, electrical isolation continues to be a problem especially due to the fact that additional medical devices (e.g. endoscopes) that are not electrically isolated are in the direct vicinity of the working area forming an electrical path from the patient to associated equipment.

There has been limited success in addressing this problem. For example, Japanese Published Application No. JP2004/202040 to Kunio et al. ("Kunio") discloses use of signal transmission between and endoscope body part and a signal processing part via an electrically non-contact transmission means such as light and a solar battery that is mounted on the endoscope body part. The light source is made incident on the solar battery via a light guide and the generated electrical power is fed to a circuit inside the endoscope body. While this system does provide a measure of electrical isolation for the power supplied to the endoscope, this still does not completely isolate the patient from electrical connection to the system as the data sent to the system for display comprises an electrical connection. Kunio therefore, only addresses part of the problem. Another disadvantage of Kunio is the fact that a solar battery is required to be positioned in the endoscope, requiring additional space, increasing weight and cost of the device. Additionally, batteries degrade over time losing effectiveness.

It is further contemplated that minimizing the placement of additional devices in the surgical area that are not electrically isolated is desirable because it will further increase patient safety.

Another problem faced by endoscopes, increasingly so for devices utilizing highly sensitive electronics is that of obtaining operating information relating to the endoscope. Operating information, including for example, the temperature that the highly sensitive electronics are exposed to, can provide valuable information for servicing and trouble-shooting of the equipment.

Accordingly, what is desired is an endoscope that electrically isolates a patient from electronics used by the endoscope system.

It is further desired to provide an endoscope that provides electrical isolation to a patient while still providing a relatively small and lightweight system with high reliability.

It is still further desired to provide an endoscope that electrically isolates a patient and reduces the number of additional devices needed to be placed in the area to be viewed.

it is yet further desired to provide an endoscope that electrically isolates a patient and provides operating information relating to the endoscope.

### SUMMARY OF THE INVENTION

These and other objects are achieved, in one advantageous embodiment, by an endoscope that includes a relatively small micro chip (digital imaging device) inserted in the endoscope that derives power from the light source via an optical channel and transmits image data generated by the digital imaging device via the optical channel to the system for display. In this manner, there is essentially no direct electrical connection between the endoscope and the patient.

It is contemplated that the digital imaging device may comprise, in an advantageous embodiment, a C-MOS chip utilizing a solar cell to transform the optical energy into electrical energy for powering of the C-MOS chip. It should be noted, however, that the digital imaging device may further comprise, for example, a CCD or other suitable image generating device as desired. The image data may further be converted into optical energy for transmission from the endoscope to the system for conversion and display for the physician. In this manner, the highest level of electrical isolation may be achieved for patient safety.

While the use of a solar cell is preferred, it is further contemplated that a solar battery may effectively be used to convert the optical energy transmitted via the light channel to electrical energy for powering the digital imaging device.

It is further contemplated that the digital imaging device may be provided with a light communication port, such as for example, an infrared port, for transmission of the image data.

It is still further contemplated that the digital imaging device may be provided with an internal thermistor, which may be used to effectively measure the temperature that digital imaging device is exposed to. The thermistor may also be used to provide actual temperature data of the area adjacent to the digital imaging device, such that additional devices and instrumentation need not be introduced into the working area.

The endoscope may also be provided with a programmable memory or storage that includes operating information relating to the endoscope including, but not limited to, endoscope type, size, age, repair history, number of uses, and number of sterilizations, etc.

In one advantageous embodiment, an endoscope system is provided comprising an imaging device for generating image data, the imaging device positioned on the endoscope and an optical channel coupling the endoscope to a camera control unit, the optical channel providing light energy to the endoscope. The endoscope system further comprises a solar cell coupled to the optical channel and the imaging device for generating electrical energy to power the imaging device and an optical port on the imaging device coupled to the light channel for transmission of the image data from the imaging device to processing circuitry for processing and display of the image data.

In another advantageous embodiment, an endoscope system is provided comprising an imaging device for generating image data, the imaging device positioned on the endoscope and an optical channel coupling the endoscope to a camera control unit, the optical channel providing light energy to the endoscope. The endoscope system further comprises a solar cell coupled to the optical channel and the imaging device for generating electrical energy to power the imaging device and a wireless data channel for transmission of the image data from the imaging device to processing circuitry for processing and display of the image data.

In still another advantageous embodiment, an endoscope system is provided comprising an imaging device for generating image data, the imaging device positioned on the endoscope and an optical channel coupling the endoscope to a camera control unit, the optical channel providing light energy to the endoscope. The endoscope system further comprises a solar cell coupled to the optical channel and the imaging device for generating electrical energy to power the imaging device. The endoscope system is provided such that the image data is coupled to the optical channel for transmission to processing circuitry for processing and display of the image data.

Other objects of the invention and its particular features and advantages will become more apparent from consideration of the following drawings and accompanying detailed description.

### BRIEF DESCRIPTION OF THE DRAWINGS

FIG. 1 is a block diagram of one advantageous embodiment of the present invention.

FIG. 2 is a block diagram according to FIG. 1.

FIG. 2A is another block diagram according to FIG. 1.

FIG. 3 is a block diagram of the optical channel of FIG. 2.

FIG. 4 is a block diagram according to FIG. 1.

### DETAILED DESCRIPTION OF THE INVENTION

Referring now to the drawings, wherein like reference numerals designate corresponding structure throughout the views.

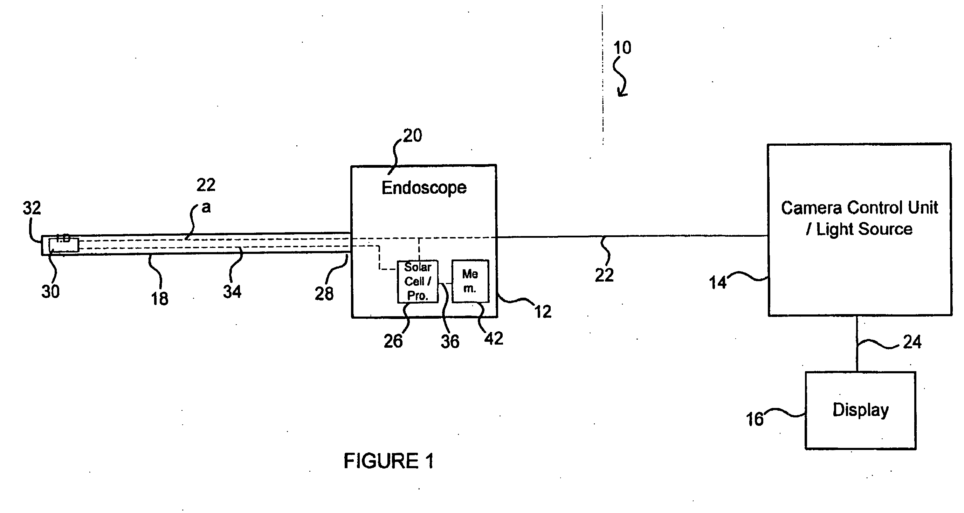

FIG. 1 is an illustration of an advantageous embodiment of system 10, which generally includes endoscope 12, camera control unit 14 and display 16. Endoscope 12 may include a shaft 18 (rigid or flexible) and a handle portion 20. Endoscope 12 is coupled to camera control unit 14 via optical channel 22, which may comprise a set of coherent optical fibers. It is contemplated that optical channel 22 may be permanently or detachably connected to endoscope 12 and/or camera control unit 14.

As indicated on FIG. 1, camera control unit 14 may be provided with a light source, which may in one advantageous embodiment, be integral with camera control unit 14.

Display 16 may comprise virtually any type of video screen display for presenting video images to the physician. The display 16 is illustrated coupled to camera control unit 14 via connection 24, which may comprise virtually any standard video cabling.

Endoscope 12 is further illustrated having a solar cell 26 positioned in handle portion 20. The solar cell 26 is coupled to optical channel 22 as is provided as a transducer to convert received optical energy into electrical energy for powering of the electronics on endoscope 12. Also noted in connection with solar cell 26 is a processor, which may be provided to process commands and data for the functioning of system 10.

Optical channel 22 is shown entering handle portion 20 of endoscope 12. Once inside endoscope 12, optical channel 22a is further illustrated as a dashed line coupled to solar cell 26 and further extends into the shaft 18 at a proximal end 28 to couple with imaging device 30 positioned at a distal end 32 of shaft 18.

In one advantageous embodiment, imaging device 30 may be a C-MOS chip embedded within the shaft 18 of endoscope 12, however, it is still further contemplated that imaging device may comprise a CCD chip or any other type of imaging chip as desired for the application.

An electrical channel 34 is further illustrated in FIG. 1 coupling solar cell 26 with imaging device 30. In this way, the optical energy received by solar cell 26 is received and transformed into electrical energy to be supplied to imaging device 30 for generation of image data.

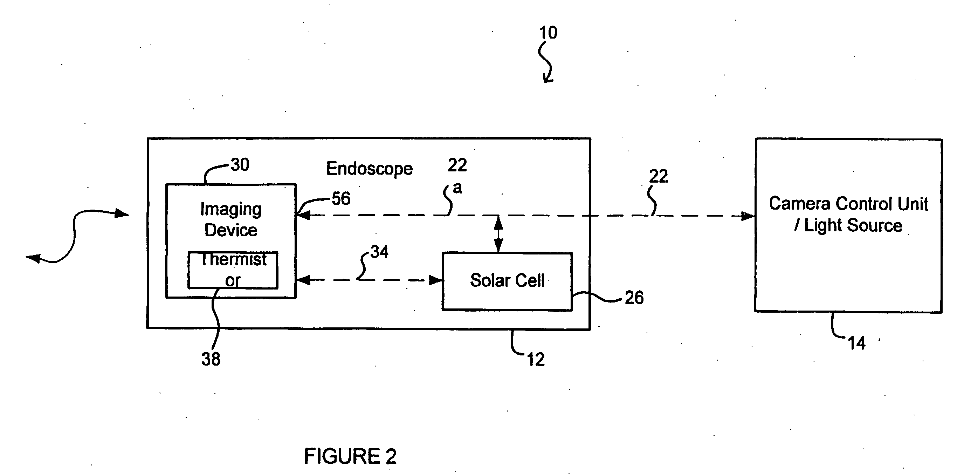

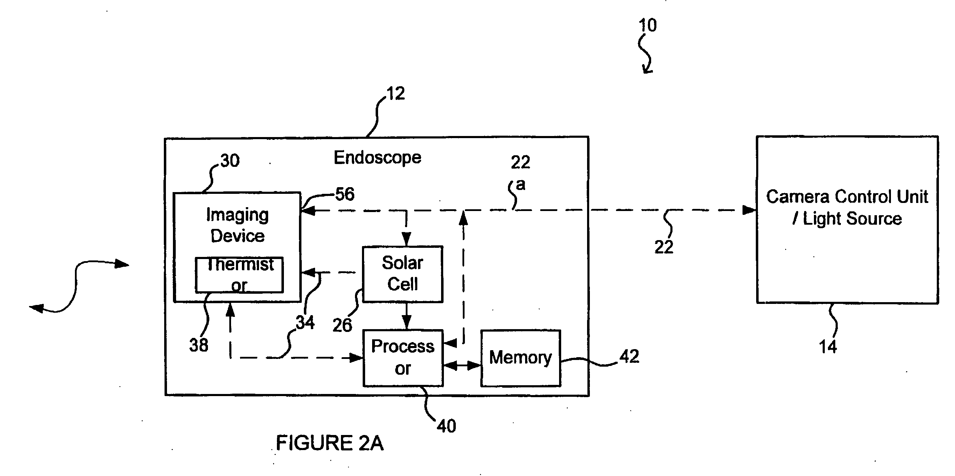

The optical channel 22a extends to the distal end 32 so as to provide illuminating light to the area ahead of the distal end 32 corresponding to the surgical area to be viewed. Light reflected from the surgical area, is picked-up by imaging device 30 and is translated into image data. The image data may, in one advantageous embodiment, be converted to optical data that is transmitted onto optical channel 22a via an optical port 56, such as for example, but not limited to an infrared port(s) (FIGS. 2-4) on imaging device 30. In this manner, the optical channel 22a, 22 serves as the illuminating light supply channel and the image data transmission channel, eliminating any electrical connection between endoscope 12 and camera control unit 14.

Alternatively, it is contemplated that the image data may be transmitted from imaging device 30 via electrical channel 34 to the processor, which in turn, may convert the image data into an optical format for transmission via optical channel 22a, 22. Again, this configuration provides excellent electrical isolation between the endoscope 12 and camera control unit 14 as only an optical channel extends therebetween.

in still another advantageous embodiment, a memory 42 is positioned in handle portion 20 of endoscope 12. The memory 42 is coupled to solar cell / processor 26 via a connection 36, which may comprise virtually any appropriate wire or group of wires as desired. It is further contemplated that, while memory 42 is show separate from solar cell / processor 26, it may be formed integral with these devices. Likewise, while solar cell / processor 26 are shown as one device, it is contemplated that these devices may be provided separate from each other or as an integrated unit. It is still further contemplated that the solar cell / processor 26 and memory 42 may be provided as a pluggable unit, which may be removed, for example during sterilization, from handle portion 20 and reinserted as desired. As a pluggable unit, the electronic devices would be provided with both an optical and an electrical connection such as is described in U.S. Patent No. 6,494,826, which is specifically incorporated herein by reference.

Memory 42 may be provided as a memory storage device for storage of various information relating to endoscope 12. For example, memory 42 may include: the type of endoscope including various information relating to the proper functioning, operation and control of the endoscope; the age of the endoscope; the repair history of the endoscope; the number of uses; and the number of sterilizations. This various information will advantageously provide the system 10 with the ability to identify the connected endoscope 12 for proper operation, while at the same time; provide specific information about to the connected endoscope relating to the actual use and maintenance of the selected endoscope. All of this information being stored in memory 42. It is further contemplated that additional information, especially related to the use of the endoscope will be written to memory 42 when the endoscope is used and/or maintained.

In this manner, a highly versatile system 10 is provided for connection of many differing types of endoscopes is provided for, while still providing for increased electrical isolation between endoscope 12 and camera control unit 14 leading to increased patient safety.

Turning now to FIG. 2, another advantageous embodiment is provided for system 10. In this embodiment, imaging device 30 is illustrated as positioned on endoscope 12 in connection with thermistor 38. Thermistor 38 is provided to generate a temperature signal and may be positioned in the vicinity of imaging device 30.

In operation, optical energy is transmitted via optical channel 22 and is received by solar cell 26 coupled thereto. The Optical channel also extends to the imaging device 30 to provide illuminating light to the area to be viewed. Solar cell 26 converts the optical energy to electrical energy, which is transmitted to imaging device 30 via electrical channel 34. Imaging device 30 picks-up reflected light from the area to be viewed, and generates corresponding image data. The image data is converted to optical energy and is transmitted to camera control unit 14 via optical channel 22a, 22. In addition, the thermistor 38 also generates a temperature data signal, which is also converted into optical energy and is transmitted to camera control unit via optical channel 22a, 22. In this manner, effective electrical isolation is achieved between the endoscope 12 and camera control unit 14.

It should be noted that, while various functions and methods have been described and presented in a sequence of steps, the sequence has been provided merely as an illustration of one advantageous embodiment, and that it is not necessary to perform these functions in the specific order illustrated. It is further contemplated that any of these steps may be moved and/or combined relative to any of the other steps. In addition, it is still further contemplated that it may be advantageous, depending upon the application, to utilize all or any portion of the functions described herein.

Referring now to FIG. 2A, another embodiment is illustrated including processor 40 and memory 42. This embodiment may function similarly to that described in connection with FIG. 2 and is further provided to receive commands signals provided by camera control unit 14 based upon the identification of endoscope 12 from the data stored in memory 34.

As an alternative embodiment, it is contemplated that imaging device 30 may supply the image data to processor 40, which is coupled to optical channel 22a, 22, such that the image data as well as endoscope information and received command signals are processed through processor 40. This embodiment also provides superior electrical isolation for the patient.

While solar cell 26, processor 40 and memory 42 are illustrated in FIG. 2A as separate devices and/or functions, it is contemplated that all or some of these items may be provided as a single integrated circuit or chip, which may advantageously be positioned on endoscope 12.

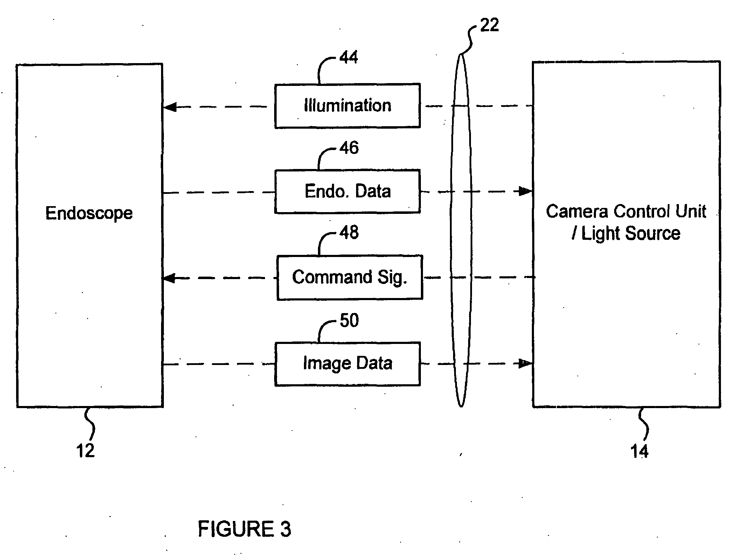

Referring now to FIG. 3 the optical channel 22 is illustrated in greater detail. As shown, optical channel 22 includes a number of channels located thereon. For example, optical channel 22 is used for transmission of illuminating light 44, which is used for illumination of the area ahead of the distal end 32 of endoscope 12. Also shown is endoscope data 46, which may be stored in memory 42 and corresponds to specific endoscope information enabling camera control unit 14 to process and control endoscope 12. Still further, command signal 48 is shown. Once the camera control unit 14 has identified the connected endoscope 12, correct command signals may then be sent for command and controlling of the endoscope 12. Finally, image data 50 is illustrated being sent over a channel on optical channel 22. In this manner, only an optical connection is provided between endoscope 12 and camera control unit 14.

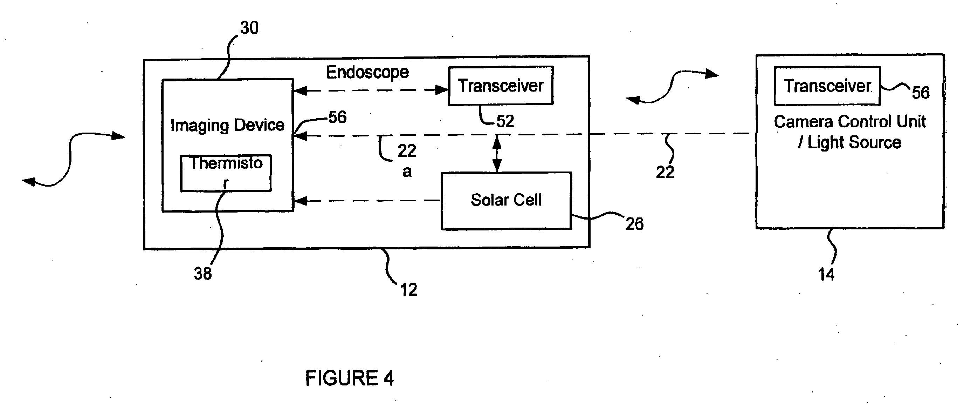

Turning now to FIG. 4, an alternative embodiment is depicted. System 10 function in a similar way as described in connection with FIG. 2, however, transceivers 52 and 54 are illustrated positioned in endoscope 12 and camera control unit 14 respectively.

In this particular embodiment, it is contemplated that the image data, as well as any endoscope data and command signals, may be wirelessly transmitted between transceiver 52 to transceiver 54. In this example, optical channel 22 is provided to supply illuminating light to imaging device 30 and electrical power may be generated by solar cell 26 to power electronics positioned in endoscope 12.

This embodiment also provides for superior electrical isolation between endoscope 12 and camera control unit 14 as only an optical and a wireless connection extends therebetween providing increased safety for the patient.

It is further contemplated that additional functionality may be provided in endoscope 12, which may be provided to transmit virtually any type of information from distal end 32 to camera control unit 14 via optical channel 22 in addition to a temperature signal. In this manner, use of multiple devices, which may or may not be electrically isolated, may be minimized thereby reducing any risk of shock to the patient.

In order to summarize, the endoscope device described above provides electrical isolation between the endoscope and a camera control unit coupled to the endoscope. The endoscope uses an optical channel to transmit illuminating light to the endoscope and to transmit image data generated by an imaging device to the camera control unit. In this manner, only an optical connection between the endoscope and the camera control unit exits. Alternatively, a wireless channel for transmission of the image data and any commands signals may be utilized.

Although the invention has been described with reference to a particular arrangement of parts, features and the like, these are not intended to exhaust all possible arrangements or features, and indeed many other modifications and variations will be ascertainable to those of skill in the art.

## Claims

1. An endoscope system comprising:
an imaging device for generating image data, said imaging device positioned on the endoscope;
an optical channel coupling the endoscope to a camera control unit, said optical channel providing light energy to the endoscope;
a solar cell coupled to said optical channel and said imaging device for generating electrical energy to power said imaging device.

2. The endoscope system of claim 1, further comprising:
an optical port on said imaging device coupled to said light channel for transmission of the image data from said imaging device to processing circuitry for processing and display of the image data.

3. The endoscope system of claim 1 or 2, wherein said imaging device is positioned at a distal end of a shaft on the endoscope.

4. The endoscope system of any one of claims 1 through 3, wherein said solar cell is positioned in a handle of the endoscope and an electrical connection extends from the solar cell to said imaging device.

5. The endoscope system of any one of claims 1 through 4, wherein said optical channel extends to a distal end of a shaft on the endoscope.

6. The endoscope system of any one of claims 1 through 5, wherein said optical channel comprises coherent optical fibers.

7. The endoscope system of any one of claims 1 through 6, further comprising a thermistor positioned on the endoscope to generate temperature measurement data.

8. The endoscope system of claim 7, wherein said thermistor is positioned in the vicinity of said imaging device.

9. The endoscope system of claim 7, wherein said thermistor is positioned at a distal end of a shaft on the endoscope.

10. The endoscope system of any one of claims 1 through 9, further comprising a memory positioned on the endoscope.

11. The endoscope system of claim 10, wherein said memory has endoscope data stored thereon selected from the group consisting of endoscope: type, size, age, repair history, number of uses, number of sterilizations and combination thereof.

12. The endoscope system of any one of claims 1 through 11, further comprising command data transmitted via said optical channel for controlling the endoscope.

13. The endoscope system of any one of claims 1 through 12, wherein said imaging device comprises a C-MOS chip.

14. The endoscope system of any one of claims 1 through 13, further comprising:
a wireless data channel for transmission of the image data from said imaging device to processing circuitry for processing and display of the image data.

15. The endoscope system of claim 14, further comprising command data transmitted via said wireless data channel for controlling the endoscope.

16. The endoscope system of claim 14 or 15, further comprising an endoscope transceiver positioned in the endoscope and coupled to said imaging device and a camera control unit transceiver positioned in the camera control unit.

17. The endoscope system of claim 16, wherein said endoscope transceiver is wirelessly coupled to said camera control unit transceiver for transmission of the image data.

18. The endoscope system of any one of claims 1 through 17, wherein said image data are coupled to said optical channel for transmission to processing circuitry for processing and display of the image data.

19. The endoscope system of any one of claims 1 through 18, further comprising a processor coupled to said solar cell and said imaging device for receiving and processing command signals.

20. The endoscope system of claim 19, wherein said processor receives and processed the image data which is transmitted via said optical channel to the camera control unit.

21. The endoscope system of claim 19 or 20, wherein said processor, said solar cell are provided as a single integrated chip positioned on said endoscope.

22. The endoscope system of any one of claims 1 through 21, further comprising a memory coupled to said processor.

23. The endoscope system of claim 22, wherein said processor, said solar cell and said memory are provided in a single integrated chip positioned on said endoscope.
